# EUROPÄISCHE PATENTSCHRIFT

(11) **EP 2 932 956 B1**
(45) Veröffentlichungstag und Bekanntmachung des Hinweises auf die Patenterteilung: **27.12.2017**
(21) Anmeldenummer: 14164635.6
(22) Anmeldetag: 14.04.2014
(51) Int. Cl.: A61K 8/11, A61K 8/73, A61Q 11/00, A61Q 19/00

(54) **Kapseln mit hoher Wirkstoffbeladung**
Capsules with high active load
Capsules avec une charge d'agent actif élevée

(43) Veröffentlichungstag der Anmeldung: 21.10.2015
(73) Patentinhaber: Symrise AG, 37603 Holzminden (DE)
(72) Erfinder: Aickele, Frank, 37603 Holzminden (DE); Weißbrodt, Jenny, 37603 Holzminden (DE)
(74) Vertreter: Fabry, Bernd

(56) Entgegenhaltungen:
- EP-A1- 1 508 591
- EP-A1- 2 801 263
- EP-A2- 1 110 462
- EP-A2- 1 295 538
- EP-A2- 2 340 805

## Beschreibung

### GEBIET DER ERFINDUNG

Die Erfindung befindet sich auf dem Gebiet von (getrockneten) Kapseln mit einer hohen Wirkstoff- bzw. Wirksubstanzbeladung, sowie deren Verwendung in kosmetischen und/oder pharmazeutischen Zusammensetzungen und die Herstellung der Kapseln.

### STAND DER TECHNIK

Verkapselungen oder Einkapselungen von Wirkstoffen, insbesondere von Aroma- bzw. Duftstoffen oder kosmetischen oder pharmazeutischen Wirkstoffen sind Stand der Technik und bietet oftmals die Möglichkeit, das verkapselte bzw. eingekapselte Material zu stabilisieren und vor Reaktionen mit dem Medium zu schützen, um so die Wirkung des Wirkstoffes zu erhalten und langsam frei zusetzen. Als Verkapselungsmaterialien gibt es unterschiedliche Möglichkeiten. Ein dem Fachmann bekanntes Matrixmaterial ist Alginat.

Alginate sind lineare Polysaccharide, deren monomere Bausteine β-D-Mannuronat und α-L-Guluronat. Die β-D-Mannuronat- und α-L-Guluronat-Reste im Alginat sind über 1,4-glykosidische Bindungen miteinander verknüpft und bilden Sequenzen mit Homopolymer- und alternierender Struktur. Der Polymerisationsgrad liegt zwischen 100 und 3000, was eine Molekülmasse von 20 000 bis 600 000u entspricht. Bei den Homopolymer-Sequenzen unterscheidet man zwei verschiedene Kategorien: Während die sogenannten "MM-Blöcke" aus β-1,4-glykosidisch verknüpften D-Mannuronat-Resten bestehen, liegen in den "GG-Blöcken" L-Guluronat-Reste mit α-1,4-glykokosidischer Bindung vor. Im dritten Alginat-Blocktyp, der Sequenz mit alternierender Struktur, sind D-Mannuronat und L-Guluronat- reste abwechselnd β-1,4- und α-1,4-glykosidisch miteinander verbunden. Folglich existieren im Alginat alle vier möglichen Arten von glykosidischen Bindungen: di-äquatorial (MM), diaxial (GG), äquatorial-axial (MG) sowie axial-äquatorial (GM).

Verfahren zur Herstellung von Calciumalginat- Partikeln sind ebenfalls seit langem Stand der Technik. Häufig wird bei der Herstellung die sogenannte Eintropfmethode angewendet. Hierbei wird eine Lösung aus Natriumalginat über eine Hohlnadel oder Kanüle in eine Lösung eines Vernetzers, z.B. Calciumsalzlösung ("ionogener Crosslinker") getropft. Zur Vermeidung von Agglomerationen können rotierende oder vibrierende Nadel oder Kanüle eingesetzt werden. Auch andere zweiwertige Kationen wie Sr2+, Ba2+, Pb2+, Cu2+, Cd2+, Co2+, Ni2+, Zn2+ und Mn2+ sind als ionogene Vernetzer geeignet. Ihr Einsatz ist jedoch aufgrund durch ihre Toxizität begrenzt. Das rein ionogene Vernetzen und das damit verbundene Gelieren der Alginatlösung wird hauptsächlich durch den Austausch der Na+-Ionen des Gluronates im Polymeren durch z.B. Ca2+ bewirkt. Die dadurch hervorgerufene besondere räumliche Anordnung der Guluronsäuresequenzen um das zweiwertige Ca2+-lon, ist in der Literatur unter dem Begriff "Egg-Box-Model" bekannt und ist verantwortlich für den Aufbau des resultierenden Gel-Netzwerkes. Dieses verleiht dem sich bildenden Partikel (Bead) die Festigkeit. Spährische Partikel aus Alginat, hergestellt durch Eintropfen einer Alginatlösung in ein Reservoir eines ionognen Crosslinkers, werden in der Literatur vielfach zum Einkapseln der verschiedensten Substanzen, z.B. Hefezellen (T. Shiotani et.al. Eur. J. Appl. Microbial. Biotechnol. 13 (2), 1981), Bakterein (H. Provost et. al. Biotechnology Letters, 7(4), 247-252, 1985) sowie Therapeutika (US 485, 471). Zur Inkorporationszwecken wird das zu einzukapselnde Material der Alginatlösung vor Kontakt mit dem Vernetzungsmedium zugesetzt.

Bei der DE 10026453 A1 (LUEKEN H) handelt es sich um magnetische und nicht magnetische sphärische Partikel, die aus einem formbeständigen Alginantgel bestehen, das durch Salzbrücken zwischen den Carboxylgruppen des Alginates vernetzt wird.Die sphärischen Partikel können inkoporiertes magnetisches Material enthalten Alginat mit einem D-mannuronate (M): L-guluronate (G)- Verhältnis von 0.1:1 bis kleiner als 1: 1, eine oder mehrere kationsiche Spezies, vorzugsweise ein zweiwertiges oder mehrwertiges Metallkation, undein oder mehrere Mittel mit günstigen Eigenschaften.

Die EP 0766515 B1 (CP KELCO) beschäftigt sich mit Alginatkapseln, in denen Wirkstostoffe enthalten sind, und verzögert freigesetzt werden. Die Alginatkapseln weisen eine Zusammensetzung auf, in denen das Gewichtsverhältnis von Alginat zu Polyacrylsäure von 75:2 bis 75:10 liegen und die Polyacrylsäure ein Molekulargewicht von 10 bis 250 kDa hat.

Die EP 1110462 A2 (NUTRINOVA) behandelt eine verkapselte biologische aktive Nahrungsmittelkomponente, die aus einem Kern, der mindestens einen von einer biologischen aktiven Substanz umgebenen Ballaststoff enthält, und einer den Kern umgebenden hüllbildenden Substanz besteht, worin der Ballaststoff Weizenfasern und Pektin, die biologische aktive Substanz Nährstoff, sowie die hüllbildende Substanz Emulgatoren darstellen können.

Demgegenüber offenbart die EP 1295538 A2 (NUTRINOVA) eine Ballaststoffkapsel, deren Kern Johannisbrotfaser enthält und allseitig durch Hüllsubstanzen umgeben ist, worin die Hüllsubstanze aus löslichen Ballaststoffe ausgewählt ist, die Alginat, Pektin, Gummi Arabikum, und modifizierte Stärke sein können.

Die EP 2099889 B1 (UNILEVER) offenbart ebenfalls Alginatkapseln (-perlen), allerdings nur für den Waschmittelbereich.

Problematisch bei den Partikeln im Stand der Technik ist, insbesondere wenn eine hohe Beladung von Aroma- oder Duftstoffe erreicht werden soll, dass die Kapseln im nassen Zustand mikrobiell instabil sind. Durch eine Trocknung der Kapseln kann eine verbesserte Stabilität erreicht werden. Jedoch werden oftmals durch den Trocknungsvorgang, die in den Kapseln eingekapselten Wirkstoffe, vor allem wenn es sich um Aroma- bzw. Duftstoffe handelt, herausgepresst und ungewollt freigesetzt, so dass die Partikel schrumpfen und so ein Teil der eingekapselten Wirkstoffe (Aroma- bzw. Duftstoffe) noch vor der eigentlichen Anwendung verloren gehen.

Die Aufgabe der vorliegenden Erfindung hat daher darin bestanden, stabile Kapseln, die Wirkstoffe bzw. Substanzen enthalten, bereitzustellen, die eine gezielte Freisetzung der eingeschlossenen Wirkstoffe bzw. Substanzen, zum Beispiel bei der Erhitzung, beim Braten und Frittieren oder bei starker Scherung, wie beim Kauen oder Verreiben, aufweisen. Somit war es Aufgabe der vorliegenden Erfindung Kapseln zu entwickeln, die nicht wasserbasiert und wasserlöslich sind, da die meisten Lebensmittel und kosmetischen Produkte Wasser enthalten und so, die geforderte Freisetzung bei der Erhitzung, beim Verzehr oder durch Scherung nicht optimal oder nur sehr begrenzt erfüllt werden.

Ferner war es Aufgabe der vorliegenden Erfindung Kapseln zu entwickeln, die eine variable Beladung ermöglichen, und auch eine hohe Wirkstoffbeladung zu lassen, so dass die Kapseln breitmöglichst angewendet werden können, beispielsweise für den kosmetischen und pharmazeutischen Bereich, insbesondere für den Oral Care Bereich oder im dermatologischen Bereich. Die Wirkstoffe und Substanzen in den Kapseln sollen auch hier wasserunlöslich sein und, erst durch Reibung bzw. Scherung, z.B. beim Zähneputzen oder durch Verreiben auf der Haut, freigesetzt werden.

### BESCHREIBUNG DER ERFINDUNG

Gegenstand der Erfindung sind Kapseln, umfassend
(a) mindestens eine gelierbare Substanz,
(b) mindestens einen Emulgator,
(c) mindestens einen Füllstoff und
(d) mindestens einen Wirkstoff, welcher verkapselt werden soll,
wobei
(i) die gelierbare Substanz (a) ausgewählt ist aus Alginat und/oder Pektin und/oder Gellan Gummi,
(ii) der Emulgator (b) ausgewählt ist aus der Gruppe bestehend aus Polysorbate, Zuckereestern, Saponine, Gummi Arabicum, modifiziertem Gummi Arabicum und/oder modifizierte Stärke,
(iii) der Füllstoff (c) ausgewählt ist aus der Gruppe bestehend aus pflanzlichen Fasern, mikrokristalline Cellulose, Silicagele, native Stärke und/oder Silikate, und
(iv) der Wirkstoff (d) in einer Emulsion umfassend die gelierbare Substanz (a), den Emulgator (b) und den Füllstoff (c) dispergiert vorliegt.

Überraschenderweise wurde gefunden, dass, wenn die Kapselmatrix aus einer Emulsion besteht, welche mindestens eine gelierbare Substanz, mindestens einen Emulgator und mindestens ein Füllstoff umfasst, Wirkstoffe bzw. Wirkstoffsubstanzen, in der Kapselmatrix fein dispergiert eingearbeitet werden kann. Dies führt zu einer hohen Wirkstoff- bzw. Wirksubstanzbeladung der Kapselmatrix mit dem gewünschten Wirkstoff bzw. Wirksubstanz und zu einer erhöhten Stabilität der Kapseln, so dass die Wirkstoffe bzw. Wirksusbtanzen in derTrocknungsphase der Kapseln nicht ausgepresst werden und ungewollt freigesetzt oder zerstört werden.

Vorzugsweise weisen die erfindungsgemäßen Kapseln eine sehr hohe Wirkstoffbeladung auf, die je nach Anwendungsbedarf variabel eingestellt wird. Bevorzugt weisen die erfindungsgemäßen Kapseln demgemäß eine Wirkstoffbeladung von 20 bis 95 Gew.% auf. Die Wirkstoff- bzw. Wirksubstanzbeladung ist abhängig von der Endapplikation der Kapseln und variiert dementsprechend je nach Einsatzgebiet. So können Kapseln, die mit Aroma oder Duftstoffen beladen sind, vorzugsweise bis zu 95 Gew.% Beladung aufweisen, wohingegen bei Kapseln, die im Bereich Medizin eingesetzt werden, die Beladung mit therapheutischen Wirkstoffen vorzugsweise bei 20-60 Gew.%, bevorzugt bei 30-50 Gew.% liegen. Im medizinischen Bereich ist die Beladung der Kapseln natürlich auch von der Dosiermenge und der erwünschten Wirkstoffmenge abhängig, die freigesetzt werden sollen. Entsprechend können dann die erfindungsgemäßen Kapseln dann beladen werden. Die Beladungsmenge kann je nach Anwendungsbedarf entsprechend variiert und eingestellt werden. Demgemäß sind die vorgenannten Beladungsmengenbereiche als mögliche Beispielgrößen zu verstehen, und sollen keine Einschränkung für die herstellbare Beladungsmengen darstellen.

Die Anwendung der erfindungsgemäßen Kapseln in Zahnpasta ist besonders vorteilhaft, da die erfindungsgemäßen Kapseln, die eingekapselten Substanzen besonders gut in der Zahnpasta stabilisieren, um diese kontrolliert erst bei der Anwendung (Zähneputzen), freisetzen bzw. abgeben. Bevorzugt handelt es sich bei den eingekapselten Wirkstoffen bzw. Wirksubstanzen um Putzkörper, Schaumbildner, Netz- und Feuchthaltemittel, Geschmacks- und Aromastoffe, Konservierungsmittel sowie Farb- und Zusatzstoffe und bei zahnmedizinischen Zahnpasta oder -cremes Wirkstoffe zur Prophylaxe, speziell von Parodontitis und Karies (Fluoride). Vorzugsweise weist eine solche Kapsel in Zahnpasta eine Beladungsmenge der Wirkstoffe bzw. Wirksubstanzen von 50 bis 80 Gew. %, bei Aromastoffen sogar bis zu 95 Gew.%, auf.

Die Anwendung der erfindungsgemäßen Kapseln in kosmetischen Produkten, insbesondere im dermatologischen Bereich, wie beispielsweise (Haut-) Cremes, Pasten, Gele und Lotionen, aber auch Sprays, sind ebenfalls besonders vorteilhaft, da die erfindungsgemäßen Kapseln, die gewünschten Substanzen, bevorzugt sind es Duft- und Aromastoffe oder kosmetische Wirksubstanzen, besonders gut stabilisieren und kontrolliert erst bei der Anwendung (durch Verreiben auf der Haut), freisetzen. Vorzugsweise weist eine solche Kapsel in kosmetischen Produkten eine Beladungsmenge der Wirkstoffe bzw. Wirksubstanzen von 40 bis 80 Gew.%, bei Duft- und Aromastoffen sogar bis zu 95 Gew.%, auf.

Vorzugsweise können die erfindungsgemäßen Kapseln einen mittleren Durchschnittsdurchmesser von 200 bis 1500 µm, bevorzugt von 400 bis 900 µm, ganz besonders bevorzugt von 500 bis 800 µm, aufweisen. Die Kapselgröße kann je nach Anwendungsbedarf entsprechend variiert und eingestellt werden. Demgemäß sind die vorgenannten Kapselgrößenbereiche als mögliche Beispielgrößen zu verstehen, und sollen keine Einschränkung für die herstellbaren Kapselgrößen darstellen.

In der vorliegenden Anmeldung wird der Begriff Kapsel mit dem Begriff Partikel gleichgestellt. Beide Begriffe sind äquivalent und untereinander austauschbar zu verstehen.

Als "gelierbare Substanzen" werden in der vorliegenden Anmeldung Verbindungen verstanden, die in Wasser (auf-)quellen oder Wasser binden, und so zu einer Gelierung führen und eine gallertartige Masse bilden.

Erfindungsgemäße geeignete gelierbare Substanzen sind ausgewählt aus der Gruppe bestehend aus Alginat, Pektin, Agar-Agar, Carrageenan, Gellan Gummi, Gelatine, modifizierte Cellulose und /oder Proteine verwendet.

Insbesondere bevorzugt sind Alginat, Pektin, Carrageenan und/oder Gellan Gummi, ganz besonders bevorzugt sind Alginat und/oder Pektin und/oder Gellan Gummi, so dass in eine bevorzugte Ausführungsform die gelierbare Substanz Alginat und/oder Pektin und/oder Gellan Gummi sind, so dass die resultierenden Kapseln Alginat basierende und/oder Pektin basierende und/oder Gellan Gummi basierende Kapseln sind, bevorzugt sind Pektin oder Alginat oder Alginat-Pektin oder Alginat-Gellan Gummi basierende Kapseln.

Vorzugsweise weisen die erfindungsgemäßen Kapseln bei einer Mischung aus Alginat und Pektin bzw. bei Alginat und Gellan Gummi, ein bevorzugtes Verhältnis von Alginat zu Pektin von 1:2 bis 2:1 auf, und bevorzugt bei Alginat zu Gellan Gummi von 3:2 bis 3:1 auf.

Alginat ist die allgemeine Bezeichnung für Alginsäure und deren Salze. Das M:G Verhältnis des verwendeten Alginats in der vorliegenden Anmeldung ist bevorzugt von 0.1:1 bis zu weniger als 1:1, beispielsweise von 0.1:1 bis 0.99:1. D.h., dass im verwendeten Alginat die Anzahl der G-Reste größer ist als die M-Reste. Bevorzugt ist ein Verhältnis von MG: von 0.1:1 bis 0.8:1, besonders bevorzugt 0.2:1 bis 0.8:1. Geeignete Algengattungen zur Gewinnung von Alginat sind beispielsweise *Laminaria, Ecklonia, Macrocystis, Lessonia, Ascophylum und Durvillea.*

Als Alginat wird vorzugsweise Natriumalginat (E 401), Kaliumalginat (E 402), Ammoniumalginat (E 403), Calciumalginat (E 404) und Propylenglycolalginat (PGA, E 405) eingesetzt. Geeignete Alginate sind unter dem Handelsnamen "Manguel" (Manguel GMB) der Firma International Speciality Products, under dem Handelsnamen "Protonal" der Firma FMC Biopolymer und unter dem Namen "Satialgine", "Cecalgum" und "Algogel" der Firma Texturant Systems erhältlich.

Die Kationenspezies zur Ausbildung einer Gelmatrix mit dem Alginat kann im Grunde jede Spezies sein, die in der Lage ist mit dem Alginat eine Gelmatrix auszubilden. Bevorzugt sind Metallkationen, insbesondere bevorzugte Kationen bilden in wässrige Lösung divalente oder polyvalente Metallsalze aus.

Erfindungsgemäß wird daher bei einer wäßrigen Lösung eines Alkali- oder Ammoniumsalzes von Alginsäuren oder Pektinsäuren, Vernetzungsmitteln verwendet, welche bevorzugt ein zweiwertiges Kation, vorzugsweise Calcium enthält. Erfindungsgemäß wird bei der Verwendung von Carrageenan ein Vernetzungsmittel mit bevorzugt Kaliumionen verwendet.

Pektine sind pflanzliche Polysaccharide, genauer Polyuronide. Die Substanzklasse der Pektine tritt in einer Vielzahl von Strukturen auf. Allen gemein ist, dass es sich hierbei um Polysaccharide handelt, deren Hauptbestandteil (zu mind. 65 Gewichts-%) die α-D-Galacturonsäure (pKa-Wert 2,9) als Monomer ist. Diese Galacturonsäure-Monomere sind über α-1,4-, meist auch zu einem geringen Anteil über β-1,4-glycosidische Bindungen miteinander verbunden und bilden so das Rückgrat des Pektinmoleküls. Dieses lineare Rückgrat wird periodisch durch 1,2-Bindungen mit α-L-Rhamnose unterbrochen. Daher ist die systematische Bezeichnung für Pektin Rhamno-Galacturonsäure.

Der Einbau von Rhamnose-Einheiten führt dazu, dass es in der formal geradlinigen Polygalacturonsäurekette zu Störungen kommt: die Ketten werden "geknickt". Die Rhamnose-Bausteine in natürlichen Pektinen wiederum tragen oligomere Seitenketten aus den Zuckern Arabinose, Galactose oder Xylose. Diese Neutralzuckerseitenketten können wiederum in Arabinane, Galactane und Arabinogalactan-I sowie Arabinogalactan-II, welches mit Proteinen verknüpft ist, allerdings oft auch zu den Hemicellulosen gezählt wird, unterteilt werden. Die Seitenketten liegen meist zwischen einer und 50 Zuckereinheiten. Bei der industriellen Gewinnung der Pektine gehen diese Seitenketten zum Großteil, jedoch insbesondere die säurelabile Arabinofuranose verloren. Die Verzweigungen in der Kette durch L-Rhamnose und ihre Seitenketten treten nicht regelmäßig auf, sondern häufen sich in den sogenannten "hairy regions". Im Gegensatz dazu heißen die linearen Teile der Kette "smooth regions".

Neben den Verzweigungen der Hauptkette finden sich weitere Merkmale des Pektinmakromoleküls. Die Hydroxygruppen am C2- oder C3-Atom der Galacturonsäureeinheiten sind zu geringen Teilen acetyliert oder durch weitere Neutralzucker wie D-Galactose, D-Xylose, L-Arabinose oder L-Rhamnose substituiert - auch hier vorwiegend in den hairy regions. Die Carboxygruppen der Polygalacturonsäure sind oft mit Methanol verestert. Der Grad der Veresterung und Acetylierung schwankt mit der Herkunft des Pektins, hat aber entscheidenden Einfluss auf die chemischen Eigenschaften. Deshalb werden Pektine anhand ihres mittleren Veresterungsgrads VE klassifiziert.

Pektine kommen in allen höheren Landpflanzen vor. Hier findet man Pektine in allen festeren Bestandteilen, beispielsweise den Stängeln, Blüten, Blättern usw. Besonders pektinreich sind Pflanzenteile mit relativ zähen/harten Bestandteilen, z. B. Citrusfrüchte oder Fruchtstände von Sonnenblumen. Der Gehalt an Pektin variiert von Pflanze zu Pflanze, so enthalten Äpfel ca. 1-1,5 %, Apfelsinen ca. 0,9-3,5 %, Citrusschalen (aus Orangen und Zitronen) ca. 30 %, Aprikosen ca. 1 %, Kirschen ca. 0,4 %, Möhren ca. 1,4 %, Orangen ca. 0,5-3,5 % und Quitten ca. 0,5 % Pektin.

Geeignete Pektine für die vorliegende Erfindung sind Apfel-, Citrus- und Grapefruit-Pektine, die beispielsweise bei der Firma Herbstreith & Fox erhältlich sind.

Geeignete gelierbare Substanzen, welche modifizierte Cellulose sind, werden vorzugsweise ausgewählt aus Methylcellulose (MC), Hydroxyethylcellulose (HEC) und Carboxymethylcellulose (CMC), Hydroxypropylmethylcellulose (HPMC), Hydroxypropylcellulose (HPC) und Ethylhydroxyethylcellulose (EHEC).

Geeignete gelierbare Substanzen, welche Stärke (derivate) sind, werden vorzugsweise ausgewählt aus z.B. Hydroxyethylstärke und Hydroxypropylstärke.

Vorzugsweise wird die gelierbare Substanz, welche bevorzugt Alginat und/oder Pektin und/oder Gellan Gummi ist, in eine Menge von 0.5 bis 3 Gew.% eingesetzt, bezogen aus die Gesamtmasse einer Kapsel. Besonders bevorzugt wird die gelierbare Substanz in eine Menge von 0.7 bis 2.0 Gew.%, besonder bevorzugt in einer Menge von 1.0 bis 1.7 Gew.%, ganz besonder bevorzugt in einer Menge von 1.1 bis 1.5 Gew.% eingesetzt, bezogen aus die Trockenmasse der Gesamtmasse einer Kapsel.

Erfindungsgemäße geeignete Emulgatoren sind ausgewählt aus der Gruppe bestehend aus Polysorbaten, Zuckereestern, Saponinen, Gummi Arabikum, modifiziertem Gummi Arabikum und/oder modifizierte Stärke.

Bevorzugt eingesetzte Polysorbate sind:
- Polysorbat 20 (Polyoxyethylen-(20)-sorbitanmonolaurat) (E 432)
- Polysorbat 21 (Polyoxyethylen-(4)-sorbitanmonolaurat)
- Polysorbat 40 (Polyoxyethylen-(20)-sorbitanmonopalmitat), (E 434)
- Polysorbat 60 (Polyoxyethylen-(20)-sorbitanmonostearat), (E 435)
- Polysorbat 61 (Polyoxyethylen-(4)-sorbitanmonostearat)
- Polysorbat 65 (Polyoxyethylen-(20)-sorbitantristearat), (E 436)
- Polysorbat 80 (Polyoxyethylen-(20)-sorbitanmonooleat), (E 433)
- Polysorbat 81 (Polyoxyethylen-(5)-sorbitanmonooleat)
- Polysorbat 85 (Polyoxyethylen-(20)-sorbitantrioleat), (Span 85)
- Polysorbat 120 (Polyoxyethylen-(20)-sorbitanmonoisostearat).

In einer bevorzugten Ausführungsform sind die Emulgatoren aus der Gruppe bestehend aus Gummi Arabikum, modifiziertem Gummi Arabikum und/oder modifizierte Stärke.

Erfindungsgemäße geeignete Füllstoffe sind ausgewählt aus der Gruppe bestehend aus pflanzlichen Fasern, mikrokristalline Cellulose, Silicagele, native Stärke und/oder Silikate. Bevorzugt werden Faserpflanzen als Füllstoffe eingesetzt.

Demgemäß besteht eine bevorzugte Ausführungsform der Erfindung darin, dass die eingesetzten Füllstoffe eine pflanzliche Faser ist, welche ausgewählt ist aus Apfelfasern, Bambusfasern, Haferfasern, Erbsenfasern, Kartoffelfasern und / oder Weizenfasern. Geeignete Fasern sind beispielsweise von der Firma JELUCEL unter den Handelsnamen JELUCEL® WF, JELUCEL® BF, JELUCEL® OF oder auch unter der Produktreihe Vitacel® der Firma JRS erhältlich. Besonders bevorzugt sind hierbei Apfelfasern, Bambusfasern, Haferfasern und/oder Weizenfasern.

Der Füllstoff wird vorzugsweise in einer Menge von 0.5 bis 5 Gew.% eingesetzt, bezogen auf die Gesamtmasse der Kapsel. Bevorzugt wird der Füllstoff in einer Menge von 0.7 bis 3.5 Gew.%, ganz besonder bevorzugt in einer Menge von 0.8 bis 1.5 Gew.% eingesetzt, bezogen auf die Gesamtmasse einer Kapsel.

Eine besondere Herausforderung bei der vorliegenden Erfindung war die Trocknung der hochbeladenen Kapseln. Es war wichtig Kapseln herzustellen, bei denen der Wirkstoff, der in der Matrix eingeschlossen vorliegt, nicht durch die Trocknung herausgepresst wird, und dadurch noch vor der Anwendung freigesetzt oder zerstört wird.

Ein besonderer Vorteil der erfindungsgemäßen Kapseln ist, dass sie sich gut trocknen lassen, ohne dass ein großer Verlust der eingeschlossenen Wirkstoffe und Wirksubstanzen (durch unkrontrollierte Freisetzung) erfolgt. Vorzugsweise weisen die erfindungsgemäßen Kapseln daher einen AW-Wert von unter oder gleich 0.8, vorzugsweise unter oder gleich 0.7, ganz besonders bevorzugt unter oder gleich 0.6, auf. Der AW-Wert ist das Maß an ungebundenem und locker gebundenem Wasser, also für die Verfügbarkeit von Wasser in Lebensmitteln oder Speisen und Zubereitungen. ¹Der AW-Wert ist damit eine wichtige Größe für die Haltbarkeit von Lebensmitteln. Je größer der AW-Wert ist, also je mehr ungebundenes Wasser ein Lebensmittel enthält, desto leichter kann es verderben. Denn das frei verfügbare Wasser ist vor allem für das Wachstum bzw. den Stoffwechsel von Mikroorganismen von entscheidender Bedeutung. Zudem ist wird es von einigen Enzymen, den so genannten Hydrolasen benötigt zur Hydrolyse, also zur Spaltung von chemischen Bindungen durch Reaktion mit Wasser benötigt.
¹ http://www.lebensmittellexikon.de/a0000420.php

Für eine erfolgreiche Trocknung der Kapseln ist die erfindungsgemäße Kombination aus Emulgator, bevorzugt lebensmittelkonforme Emulgatoren, und Füllstoffen bestimmend.

Ein Verhältnis von Emulgator zu Füllstoff liegt vorzugsweise im Bereich von 2.1:1.2, besonders bevorzugt im Bereich von 1:1.

Der Einsatz der genannten Füllstoffe hat insbesondere den Vorteil, dass es die Trockenmasse in der Kapsel erhöht und den Wirkstoff bzw. die Wirksubstanz, welche in der Matrix eingekapselt vorliegt, bindet, und so bei der Trocknung stabilisiert. Daher ist der Füllstoff ein essentieller Bestandteil der vorliegenden Erfindung.

Je nach Anforderung der Endapplikation, kann die Kapseleigenschaften durch die Auswahl und Menge des Emulgators und des Füllstoffes gesteuert werden, und so Einfluss auf die Eigenschaften, wie Freisetzungsverhalten des Wirkstoffes, Stabilität der Kapsel und Beladungsmenge, genommen werden.

Ein weiterer Vorteil der erfindungsgemäßen Kapseln ist ihre Stabilität, die es ermöglicht, die Kapseln in den unterschiedlichsten Bereichen einzusetzen, um die gewünschten, je nach Anwendungsbedarf, unterschiedlichen Wirkstoffe und Wirkstoffsubstanzen in das entsprechende Medium einzubringen und bei Bedarf kontrolliert freizusetzen.

Demgemäß besteht eine weitere bevorzugte Ausführungsform der vorliegenden Erfindung in Kapseln, die Wirkstoffe bzw. Wirkstoffsubstanzen umfassen, die ausgewählt sind aus der Gruppe bestehend aus Aromastoffen, Duftstoffen, Nahrungsergänzungsmittelsubstanzen, wie beispielsweise Vitamine, Mineralstoffe, Antioxidantien, Anthocyane, Coenzym10 etc., sowie kosmetischen Wirksubstanzen und / oder pharmazeutischen Wirksubstanzen.

Ferner besteht eine bevorzugte Ausführungsform der Erfindung in den erfindungsgemäßen Kapseln zur Verwendung in kosmetischen Produkten, pharmazeutischen Mitteln oder in Nahrungs- und Genussmitteln.

Ein weiterer Gegenstand der Erfindung ist die Herstellung der erfindungsgemäßen Kapseln. Das Verfahren zur Herstellung der erfindungsgemäßen Kapseln, umfasst
(i) das Bilden einer Mischung aus
   (a) mindestens einer gelierbaren Substanz,
   (b) mindestens einem Emulgator,
   (c) mindestens einem Füllstoff und
   (d) mindesten einem Wirkstoff oder einer Wirksubstanz,
(ii) das Einbringen von Tropfen der Mischung aus i) in eine Lösung aus einem multivalenten Kation, so dass eine Vernetzung stattfindet und eine Gelierung erfolgt,
(iii) die Trennung der gebildeten Kapseln aus Schritt ii),
(iv) das Trocknen der erhaltenen Kapseln aus Schritt iii).

Fernere besteht ein weitere Gegenstand der vorliegenden Erfindung in Kapseln, welche erhältlich sind durch die Schritte, umfassend:
(i) das Bilden einer Mischung aus a) mindestens einer gelierbaren Substanz, b) mindestens einem Emulgator, c) mindestens einem Füllstoffen und d) mindesten einem Wirkstoff oder Wirksubstanz,
(ii) das Einbringen von Tropfen der Mischung aus i) in eine Lösung aus einem multivalenten Kation, so dass eine Vernetzung stattfindet und eine Gelierung erfolgt,
(iii) die Trennung der gebildeten Kapseln aus Schritt ii),
(iv) das Trocknen der erhaltenen Kapseln aus Schritt iv), wobei die gelierbare Substanz a) ausgewählt ist aus der der Gruppe bestehend aus Alginat,Pektin, Agar-Agar, Carrageenan, Gellan Gummi, Gelatine, modifizierte Cellulose und/oder Proteine, und der Emulgator b) ausgewählt ist aus der Gruppe bestehend aus Polysorbate, Zuckereestern, Saponine, Gummi arabicum und/oder modifizierte Stärke, und c) der Füllstoff ausgewählt ist aus der Gruppe bestehend aus pflanzlichen Fasern, mikrokristalline Cellulose, Silicagele, native Stärke und/oder Silikate.

Erfindungsgemäß wird eine Emulsion aus a) mindestens einer gelierbaren Substanz, b) mindestens einem Emulgator, c) mindestens einem Füllstoff und d) mindesten einem Wirkstoff oder einer Wirksubstanz hergestellt. Aus der Emulsion werden anschließend diskrete Tröpfchen erzeugt, die in einem Zellbad aus mehrwertigen Kationen eingebracht werden, so dass formbeständige, im wesentlich wasserunlösliche Kapseln entstehen. Vorzugsweise wird eine alkoholische Lösung, die bevorzugt Calciumionen enthält, verwendet.

Bei der Erzeugung der Tröpfchen, können durch die Dimensionen der verwendete Nadeln/Kanüle die Partikelgröß eingestellt werden. Die Verwendung von rotierender oder vibrierender Nadeln/Kanülen ist ebenfalls vorteilhaft, da dadurch die Tröpfchen besser abgeschert werden. Ebenso vorteilhaft ist die Verwendung eines beweglichen Fällungsbades, so dass die gebildeten Tropfen weiter transportiert werden, und die Tropfen nicht an einer Stelle agglomerieren können.

Die so erhältlichen Kapseln können zur Entfernung von Salzresten mit Wasser abgespült werden.

Die Trocknung der Kapseln erfolgt mittels Wirbelschichtrocknung, bei der die zu trocknenden Kapseln vorzgsweise von unten mit warmer Luft durchströmt und so in der Schwebe gehalten und durchmischt und getrocknet wird.

Vorzugsweise wird das Feuchtgut im aufwärts gerichteten, heißen Gasstrom turbulent vermischt und kann dadurch bei hohen Wärme- und Stoffübergangskoeffizienten trocknen. Die erforderliche Gasgeschwindigkeit hängt vorzugsweise im Wesentlichen von der Partikelgöße und -dichte ab. Die Vewendung von perforierten Boden (Lochblech, Conidur - Blech, Böden aus Gewebe oder Sintermetall) kann vorzugsweise das Durchfallen des Feststoffs in den Heißgas - Raum verhindern. Die Wärmezufuhr erfolgt bevorzugt entweder über das Trocknungsgas oder es können vorzugsweise zusätzliche Wärmetauscher (Rohrbündel oder Platten) in die Wirbelschicht eingebracht werden.

Die für die Trocknung verwendeten Wirbelschichttrockner können sowohl kontinuierlich als auch batchweise betrieben werden. Beim kontinuierlichen Betrieb beträgt die Verweilzeit im Trockner bevorzugt mehrer Minuten bis zu Stunden; der Apparat ist deshalb auch zur Langzeit - Trocknung geeignet. Wenn eine enge Verweilzeitverteilung erforderlich ist kann die Wirbelschicht vorzugsweise durch Trennbleche kaskadiert werden oder der Produktfluß wird durch meanderförmige Einbauten einer idealen Kolbenströmung angenähert. Insbesondere größere Trockner werden bevorzugt in mehrere Trocknungszonen unterteilt, die mit unterschiedlicher Gasgeschwindigkeit und -temperatur betrieben werden. Die letzte Zone kann dann als Kühlzone genutzt werden. Im Aufgabebereich des Feuchtguts ist darauf zu achten daß keine Verklumpungen auftreten. Dazu gibt es verschiedene Möglichkeiten, z.B. eine lokal höhere Gasgeschwindigkeit oder ein Rührwerk.

Bevorzugt wird in einem solchen Verfahren Alginat und/oder Pektin und/oder Gellan Gumi basierende Kapseln hergestellt. Die erfindungsgemäßen Kaspeln sind vorzugsweise wasserunlöslich und stabil. Die Kapselwandung der erfindungsgemäßen Kapseln besteht aus einer Matrix aus einem wasserunlöslichen, mehrwertigen Kation, vorzugsweise aus Calcliumionen, und in der Matrix eingebettet sind Füllstoff und Wirkstoff bzw. Wirksubstanzen. Die erfindungegmäßen Kapslen weisen vorzugsweise keine typische Kern-Hüllen-Struktur auf, in der im Kern der Wirkstoff bzw. die Wirksubstanz eingeschlossen vorliegt, und von einer Hülle eingeschlossen wird. Vielmehr ist es so, dass die Wirkstoffe bzw. Wirksubstanzen in der Kapselmatrix verteilt eingebettet vorliegen.

In einer bevorzugten Ausführungsform sind die erfindungsgemäßen Kapseln besonders geeignet zur Verwendung in kosmetischen Produkten, pharmazeutischen Mitteln oder in Nahrungs- und Genussmittel.

Zudem besteht ein weiterer Gegenstand der vorliegenden Erfindung in der Verwendung der erfindungsgemäßen Kapseln zur Herstellung von kosmetischen Produkten, pharmazeutischen Mitteln oder Nahrungs- und Genussmitteln.

Ferner besteht ein weiterer Gegenstand der vorliegenden Erfindung in der Verwendung der erfindungsgemäßen Kapseln zur Herstellung von pharmazeutischen Mitteln oder kosmetischen Produkten zur Anwendung auf der Haut oder zur oralen Verwendung.

Insbesondere sind die erfindungsgemäßen Kapseln dazu geeignet, Wirkstoff und Wirkstoffsubstanzen in der Matrix einzuschließen, um diese später kontrolliert freizusetzen. Demgemäß besteht eine bevorzugte Ausführungsform darin, dass die erfindungsgemäßen Kaspeln zur kontrollierten, verlängerten und verzögerten Abgabe bzw. Freisetzung von Wirkstoffen und Wirksubstanzen verwendet wird.

Bevorzugt werden die erfindungsgemäßen Kaspeln zur Herstellung von pharmazeutischen Mitteln oder kosmetischen Produkten verwendet, die insbesondere zur Anwendung auf der Haut geeignet sind. Bevorzugt sind hierbei sowohl kosmetische Produkte als auch pharmazeutische Mittel in Form von Salben, Cremes, Lotionen, Gele und Pasten und Sprays.

Vorzugsweise ist unter eine Salbe, Creme, Lotion, Gel und Paste eine halbfeste streichfähige Zubereitung zu verstehen, die zum Auftragen auf die Haut geeignet sind.

Solche Zubereitungen können beispielsweise auf Basis von einer wässrigen (hydrophilen) und einer öligen bzw. fetten (lipophilen) Komponente bestehen, von der die eine emulsionsartig in der anderen verteilt ist.

Es können ebenfalls hydrophile Cremes vom O/W-Typ oder lipophile Cremes vom W/O-Typ sein. Daneben gibt es Cremes, die weder eindeutig dem O/W- noch dem W/O-Typ zuordenbar sind, die aus gelartig, kohärent ineinander verteilter lipophiler und hydrophiler Phase bestehen (amphiphile Creme). Auch Strukturen einer Mehrfachemulsion vom Typ W/O/W-Emulsion sind möglich. Hier liegt die innere Phase wiederum in Form einer Emulsion vor. In die innere Ölphase sind nochmals kleinste Wassertröpfchen eingelagert. Dieser Emulsionstyp soll die Vorteile von W/O-Emulsionen und O/W-Emulsionen in sich vereinen.

Weitere Zubereitungen sind bevorzugt Salben, die in der Regel eine halbfeste und homogen aussehende Zubereitung ist, und die geeigent ist zur Anwendung auf der Haut (z. B. als Wundsalbe) oder auf den Schleimhäuten. Salben dienen zumeist der lokalen Wirkstoffapplikation oder der Pflege und dem Schutz der Haut oder Schleimhäute. Vorzugsweise besteht eine Salbe aus einer hydrophoben oder hydrophilen Grundlage aus natürlichen oder synthetischen Stoffen und kann ein einphasiges (z. B. Vaseline) oder mehrphasiges (z.B. Wasser-in-Öl) System sein.

Eine weitere bevorzugte Zubereitung ist das Gel. Gele können als viskoelastische Fluide beschrieben werden. Ihre Fluideigenschaften liegen somit zwischen denen einer idealen Flüssigkeit und denen eines idealen Feststoffkörpers. Ein Gel ist ein feindisperses System aus mindestens einer festen und einer flüssigen Phase. Die feste Phase bildet dabei ein schwammartiges, dreidimensionales Netzwerk, dessen Poren durch eine Flüssigkeit (Lyogel) oder ein Gas (Xerogel) ausgefüllt sind. Ist das Netzwerk hochporös und Luft das eingelagerte Gas, so wird das Gel auch als Aerogel bezeichnet. Beide Phasen durchdringen sich dabei vollständig (bikohärent).

Bevorzugt sind ebenfalls Zubereitungen in Form von Pasten. Eine Paste ist ein Feststoff-Flüssigkeitsgemisch (Suspension) mit einem hohen Gehalt an Festkörpern. Pasten sind vorzugsweise nicht mehr fließfähig, sondern streichfest. Beispielsweise ist eine Paste eine Salbe-Pulver-Mischung und insbesondere halbfeste Arzneiform mit hohem Gehalt an dispergierten Feststoffen ("Suspensionssalbe" mit einem Feststoffanteil von 30 Gew.%-zum Beispiel ist eine solche Paste die Pasta Zinci (Zinksalbe)).

Ein Beispiel, bei dem ein Produkt in den unterschiedlichen Zubereitungsformen vorliegen kann, ist beispielsweise die Zahnpasta (oder- creme oder-gel), die sowohl im medizinischen als auch im kosmetischen Bereich angewendet werden kann, und somit eine breite Produktpalette ist. Der Hauptbestandteil sind Putzkörper, Schaumbildner, Netz- und Feuchthaltemittel, Geschmacks- und Aromastoffe, Konservierungsmittel sowie Farb- und Zusatzstoffe. Außerdem enthalten Zahncremes auch Wirkstoffe zur zahnmedizinischen Prophylaxe, speziell von Parodontitis und Karies (Fluoride).

Erfindungsgemäß können in den oben beschriebenen Zubereitungen (Salben, Cremes, Lotionen, Gele und Pasten) Wirkstoffe und Wirkstoffsubstanzen besonders gut durch die erfindungsgemäßen Kaspeln eingearbeitet werden, da die Kapseln wasserunslöslich und so in der Grundzubereitung stabil sind und erst durch die Anwendung, vorzugsweise durch mechanische Scherung, wie beispielsweise durch das Verreiben auf der Haut oder Putzen von Zähnen, zerstört werden und die Wirkstoffe bzw. Wirksubstanzen freisetzen bzw. abgeben.

Die erfindungsgemäßen Kapseln sind insbesondere geeignet für den Einsatz oder Einarbeitung im wässerigen/ wasserbasierten Medium, da die Kapseln durch das Umgebungswasser aufweicht und leicht anquillt. Dieses Phänomen bewirkt, dass die Kapseln in der Anwendung, insbesondere in Salben, Cremes, Lotionen, Gele und Pasten, ein sehr gutes Verreiben und Verstreichen auf der Haut und dadurch die kontrollierte Freisetzung der Wirkstoffe und Wirksubstanzen ermöglichen.

Ebenfalls ist es möglich die erfindungsgenäßen Kapseln in Nahrungs- und Genussmitteln einzusetzen. Dabei sind speziell Nahrungs- und Genussmittel im Fokus, bei denen bestimmte Stoffe eingekapselt werden sollen, die später bei der Anwendung freigesetzt werden sollen.

Vorzugsweise handelt es sich bei den Nahrungs- und Genussmitteln um streichbare Genussmittel wie zum Beispiel Brotaufstrich, Fleisch-Paste, aber auch um Backwaren, beispielsweise Brot, Trockenkekse, Kuchen, Waffeln sonstiges Gebäck, Süßwaren (beispielsweise Schokoladen, Schokoladenriegelprodukte, sonstige Riegelprodukte, Fruchtgummi, Hart- und Weichkaramellen, Kaugummi), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Margarine, Mayonnaise, Remoulade, Dressings), Milchprodukte (beispielsweise Joghurt, Cremes, Aufstrich), Feinkostprodukte, wie beispielsweise Geflügelsalate.

Insbesondere bevorzugt sind hier Snacks, Backwaren, Margarine, Aufstrich und Süßwaren, wie beispielsweise Kaugummis und Fruchgummis.

Vorzugsweise werden in den erfindungsgemäßen Kapseln, die im Bereich Nahrungs- und Genussmitteln eingesetzt werden, Aromen eingekapselt, um beim Verzehr einen sogenannten "flavour burst effect" zu bewirken. So können beispielsweise fruchtige Aromen wie Orangen-, Erdbeer- oder Apfelaromen usw. in den erfindungsgemäßen Kapseln eingekapselt werden und beispielsweise diese Kapseln in Geflügelsalat, Schokolade und Eis eingearbeitet werden, so dass beim Kauen ein überraschender Effekt erfolgt. Ein besonderes Geschmackserlebnis wird auch erreicht, wenn beispielsweise die aromahaltigen erfindungsgemäßen Kapseln in Eiswaffeln oder Kekse oder dergleichen eingebacken werden, so dass beim Verzehr ein überraschender Aromeneffekt erzielt werden kann.

### KAUGUMMIS

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### KOSMETISCHE UND/ODER PHARMAZEUTISCHE MITTEL

Die erfindungsgemäßen Kapseln sind insbesondere geeignet, um Wirkstoffe und Substanzen in kosmetischen Produkten und/oder pharmazeutischen Mitteln einzubringen.

Vorzugsweise können therapeutische Wirkstoffe in den erfindungsgemäßen Kapseln eingekaspelt werden. Vorzugsweise umfassen kosmteische Produkte und pharmazeutische Mittel eine Reihe von Hilfs- und Zusatzstoffe. Diese Hilfs- und Zusatzstoffe können, je nach Notwendigkeit, auch in die erfindungsgemäßen Kapseln eingekapselt werden. Die typischen Hilfs- und Zusatzstoffe, die in kosmetischen Produkte und/oder pharmazeutischen Mittel enthalten sein können und, die auch in den erfindungsgemäßen Kapseln verkapselt werden können, sind beispielsweise milde Tenside, Ölkörper, Emulgatoren, Perlglanzwachse, Kühlstoffe, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen.

Insbesondere Wirkstoffe, wie Kühlwirkstoffe sind geeignet, um in die erfindungsgemäßen Kapseln eingekapselt zu werden. Vorteilhaft ist bei einer solchen Einkapselung, dass die Kühlung, z.B. bei Cremes, Pasten, Sprays etc. erst bei der Anwendung, also beim Verreiben auf der Haut, einsetzt. Bei einem solchen Einsatz, z.B. als After-Sun Creme oder After-Sun Sprays sind die erfindungsgemäßen Kapseln besonders geeignet.

### TENSIDE

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### ÖLKÖRPER

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, vezweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### EMULGATOREN

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;
- Polyalkylenglycole sowie
- Glycerincarbonat.

Im Folgenden werden besonders geeignete Emulgatoren näher erläutert:

**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Subtrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride**. Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester**. Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Polyglycerinester.** Typische Beispiele für geeignete Polyglycerinester sind Polyglyceryl-2-Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

**Amphotere und kationische Emulgatoren**. Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C- Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### FETTE UND WACHSE

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachs, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### PERLGLANZWACHSE

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### KÜHLSTOFFE

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS² 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
² FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

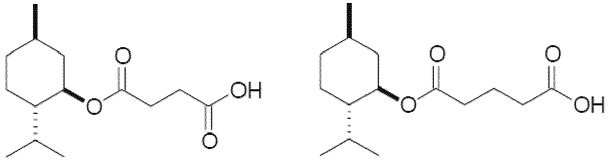

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® M PC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### KONSISTENZGEBER UND VERDICKUNGSMITTEL

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### ÜBERFETTUNGSMITTEL UND STABILISATOREN

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### POLYMERE

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/ Maleinsäurean-hydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### SILIKONVERBINDUNGEN

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-LICHTSCHUTZFAKTOREN

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.
Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### FEUCHTHALTEMITTEL

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### BIOGENE WIRKSTOFFE UND ANTIOXIDANTIEN

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### DEODORANTIEN UND KEIMHEMMENDE MITTEL

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren**. Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oderphosphate, wie beispielsweise Lanosterin-, Cholesterin-, Campesterin-, Stigmasterin-und Sitosterinsulfat bzw-phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber**. Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### FILMBILDNER

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### ANTISCHUPPENWIRKSTOFFE

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### QUELLMITTEL

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### INSEKTENREPELLENTIEN

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### INHALTSSTOFFE FÜR MUND- UND ZAHNPFLEGEMITTEL

Unter Mund- und Zahnpflegemittel sind Produkte zu verstehen, die der Mund- und Zahnreinigung und-pflege dienen. Beispiele hierfür sind Zahnpasten, Zahngele, und dergleichen.

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- vuundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### HYDROTROPE

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### KONSERVIERUNGSMITTEL

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### PARFÜMÖLE, AROMEN, AROMASTOFFE, DUFTSTOFFE

Bevorzugt eingesetzte Duftstoffe bzw. Parfümöle, sind keinerlei Beschränkungen unterworfen. So können als Duftstoffe einzelne Riechstoffverbindungen, sowohl synthetische oder natürliche Verbindungen vom Typ der Ester, Ether, Aldehyde, Ketone, Alkohole, Kohlenwasserstoffe, Säuren, Kohlensäureester, aromatische Kohlenwasserstoffe, aliphatische Kohlenwasserstoffe, gesättigte und/oder ungesättigte Kohlenwasserstoffe und Mischungen daraus verwendet werden. Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschtem Duft oder ein Frischeempfinden verleihen können. Es können auch Gemische unterschiedlicher Ketone eingesetzt werden. Beispielsweise kann das Keton ausgewählt sein aus der Gruppe, bestehend aus Buccoxim, Iso jasmon, Methyl beta naphthyl keton, Moschus indanon, Tonalid/Moschus plus, Alpha-Damascon, Beta-Damascon, Delta-Damascon,lso-Damascon, Damascenon, Damarose, Methyldihydrojasmonat, Menthon, Carvon, Campher, Fenchon, Alphalonen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenyl-keton oder Methyl-cedrylon,Acetophenon, Methylacetophenon, Para-Methoxy-acetophenon, Methyl-beta-naphtyl-keton,Benzyl-aceton, Benzophenon, Para-Hydroxy-phenyl-butanon, Celery Keton oder Livescon,6-Isopropyldecahydro-2-naphton, Dimethyl-octenon, Freskomenth, 4-(I-Ethoxyvinyl)-3,3,5,5,-tetramethyl-cyclohexanon,Methyl-heptenon, 2-(2-(4-Methyl-3-cyclohexen-1-yl)propyl)-cyclopentanon, 1-(p-Menthen-6(2)-yl)-1-propanon,4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon,4-Damascol, Dulcinyl or Cassion, Gelson, Hexalon, Isocyclemon E, Methylcyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedionund Gemischen davon. Bevorzugt können die Ketone ausgewählt sein aus AlphaDamascon, Delta Damascon, Iso Damascon, Carvon, Gamma-Methyl-ionon, Iso-E-Super,2,4,4,7-Tetramethyl-oct-6-en-3-on, Benzylaceton, Beta Damascon, Damascenon, Methyldihydrojasmonat, Methyl-cedrylon, Hedion und Gemischen davon.

Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Anisaldehyd, Cymal,Ethylvanillin, Florhydral, Floralozon, Helional, Heliotropin, Hydroxycitronellal, Koavon, Laurinaldehyd, Canthoxal, Lyral, Lilial, Adoxal, Anisaldehyd, Cumal Methyl-nonyl-acetaldehyd, Citronellal, Citronellyloxy-acetaldehyd, Cyclamenaldehyd, Bourgeonal, p,t-Bucinal, Phenylacetaldehyd, Undecylenaldehyd, Vanillin; 2,6,10-Trimethyl-9-undecenal, 3-Dodecen-I-al,alpha-n-Amylzimtaldehyd, 4-Methoxybenzaldehyd, Benzaldehyd, 3-(4-tert-Butylphenyl)-propanal,2-Methyl-3-(para-methoxyphenylpropanal), 2-Methyl-4-(2,6,6-trimethyl-2(1)-cyclohexen-1-yl)butanal,3-Phenyl-2-propenal, cis-/trans-3,7-Dimethyl-2,6-octadien-I-al, 3,7-Dimethyl-6-octen-I-al,[(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd,2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2. 1.0 (2,6)]-decylidene-8)-butanal;Octahydro-4,7-methano-IH-indenecarboxaldehyd; 3-Ethoxy-4-hydroxybenzaldehyd, para-Ethyl-alpha,alpha-dimethylhydrozimtaldehyd, alpha-Methyl-3,4-(methylenedioxy)-hydrozimtaldehyd,3,4-Methylenedioxybenzaldehyd, alpha-n-Hexylzimtaldehyd, m-Cymene-7-carboxaldehyd,alpha-Methylphenylacetaldehyd, 7-Hydroxy-3,7-dimethyl octanal, Undecenal, 2,4,6-Trimethyl-3-cyclohexene-I-carboxaldehyd,4-(3)(4-Methyl-3-pentenyl)-3-cyclohexen-carboxaldehyd, 1-Dodecanal, 2,4-Dimethylcyclohexene-3-carboxaldehyd,4-(4-Hydroxy-4-methyl pentyl)-3-cylohexene-I-carboxaldehyd, 7-Methoxy-3,7-dimethyloctan-1-al,2-Methyl undecanal, 2-Methyl decanal, 1-nonanal, 1-Octanal, 2,6,10-Trimethyl-5,9-undecadienal,2-Methyl-3-(4-tertbutyl)propanal, 3-(4-Ethylphenyl)-2,2-Dimethylpropanal, 3-(4-Methoxyphenyl)-2-methylpropanal,Methylnonylacetaldehyd, 2-Phenylpropan-1-al, 3-Phenylprop-2-en-1-al, 3-Phenyl-2-pentylprop-2-en-1-al,3-Phenyl-2-hexylprop-2-enal, 3-(4-Isopropylphenyl)-2-methylpropan-1-al, 3-(4-Ethylphenyl)-2,2-dimethylpropan-1-al,3-(4-tert-Butylphenyl)-2-methyl-propanal, 3-(3,4-Methylendioxy-phenyl)-2-methylpropan-1-al,3-(4-Ethylphenyl)-2,2-dimethylpropanal, 3-(3-Isopropylphenyl)-butan-1-al, 2,6-Dimethylhept-5-en-1-al,Dihydrozimtaldehyd, 1-methyl-4-(4-methyl-3-pentenyl)-3-cyclohexene-1-carboxaldehyd,5 oder 6 Methoxyhexahydro-4,7-methanoindan-1 oder 2-carboxyaldehyd, 3,7-Dimethyloctan-1-al,1-Undecanal,10-Undecen-1-al, 4-Hydroxy-3-methoxybenzaldehyd, 1-Methyl-3-(4-methylpentyl)-3-cyclohexene-carboxyaldehyd,7-Hydroxy-3,7-dimethyl-octanal; trans-4-decenal, 2,6-Nonadienal, para-Tolylacetaldehyd;4-Methylphenylacetaldehyd, 2-Methyl-4-(2,6,6-trimethyl-1-cyclohexen-1-yl)-2-butenal,ortho-Methoxyzimtaldehyd, 3,5,6-Trimethyl-3-cyclohexenecarboxaldehyd, 3,7-Dimethyl-2-methylene-6-octenal,Phenoxyacetaldehyd; 5,9-Dimethyl-4,8-decadienal, Peony aldehyd (6,10-dimethyl-3-oxa-5,9-undecadien-1-al),Hexahydro-4,7-methanoindan-1-carboxaldehyd, Octanal, 2-Methyl octanal, alpha-Methyl-4-(I-methylethyl)benzeneacetaldehyd,6,6-Dimethyl-2-norpinene-2-propionaldehyd, para Methyl phenoxy acetaldehyd, 2-methyl-3-phenyl-2-propen-1-al,3,5,5-Trimethylhexanal, Hexahydro-8,8-dimethyl-2-naphthaldehyd, 3-Propyl-bicyclo[2.2.1]-hept-5-ene-2-carbaldehyd,9-Decenal, 3-Methyl-5-phenyl-1-pentanal, Methylnonyl acetaldehyd, 1-p-Menthene-q-carboxaldehyd,Citral oder Gemische davon, Lilial citral, 1-Decanal, n-Undecanal, n-Dodecanal, Florhydral, 2,4-Dimethyl-3-cyclohexen-1-carboxaldehyd 4-Methoxybenzaldehyde, 3-Methoxy-4-hydroxybenzaldehyde,3-Ethoxy-4-hydroxybenzaldehyde, 3,4-Methylendioxybenzaldehyd und 3,4-Dimethoxybenzaldehydund Gemische davon. Wie vorstehend beispielhaft ausgeführt, können die Duftaldehyde und Duftketone eine aliphatische, cycloaliphatische, aromatische, ethylenisch ungesättigte Struktur oder eine Kombination dieser Strukturen aufweisen. Es können fernerweitere Heteroatome oder polycyclische Strukturen vorliegen. Die Strukturen können geeignete Substituenten wie Hydroxyl- oder Aminogruppen aufweisen. Für weitere geeignete Duftstoffe, ausgewählt aus Aldehyden und Ketonen, wird auf Steffen Arctander "Published 1960 and 1969 respectively, Reprinted2000 ISBN: Aroma Chemicals Vol. 1: 0-931710-37-5, Aroma Chemicals Vol. 2: 0-931710-38-3", verwiesen.

Geeignete Riechstoffverbindungen vom Typ der Ester sind beispielsweise Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Riechstoffverbindungen vom Typ der Kohlenwasserstoffen sind z.B. Terpene wie Limonen und Pinen. Geeignete Duftstoffe vom Typ Ether sind beispielsweise Benzylethylether und Ambroxan. Geeignete Duftstoffalkohole sind beispielsweise 10-Undecen-1-ol, 2,6-Dimethylheptan-2-ol, 2-Methylbutanol, 2-Methylpentanol,2-Phenoxyethanol, 2-Phenylpropanol, 2-tert-Butycyclohexanol, 3,5,5-Trimethylcyclohexanol,3-Hexanol, 3-Methyl-5-phenylpentanol, 3-Octanol, 1-Octen-3-ol, 3-Phenylpropanol,4-Heptenol, 4-lsopropylcyclohexanol, 4-tert-Butycyclohexanol, 6,8-Dimethyl-2-nonanol,6-Nonen-1-ol, 9-Decen-1-ol, alpha-Methylbenzylalkohol, alpha-Terpineol, Amylsa-licylat,Benzylalkohol, Benzylsalicylat, beta-Terpineol, Butylsalicylat,Citronellol, Cyclohexylsalicylat, Decanol, Dihydromyrcenol, Dimethylbenzylcarbinol,Dimethylheptanol, Dimethyloctanol, Ethylsalicylat, Ethylvanilin, Anethol, Eugenol,Geraniol, Heptanol, Hexylsalicylat, Isoborneol, Isoeugenol, Isopulegol, Linalool,Menthol, Myrtenol, n-Hexanol, Nerol, Nonanol, Octanol, para-Menthan-7-ol, Phenylethylalkohol,Phenol, Phenylsalicylat, Tetrahydrogeraniol, Tetrahydrolinalool, Thymol, trans-2-cis-6-Nonadienol,trans-2-Nonen-1-ol, trans-2-Octenol, Undecanol, Vanillin, Zimtalkohol, wobei, wenn mehrere Duftstoffalkohole vorhanden sind, diese unabhängig voneinander ausgewählt sein können.

Duftstoffe bzw. Parfümöle können auch natürliche Riechstoffgemische sein, wie sie aus pflanzlichen Quellen zugänglich sind, z.B. Pine-, Citrus-, Jasmin-, Patchouly-, Rosen- oder Ylang-Ylang-öl. Ebenfallsgeeignet sind Muskateller-Salbeiöl, Kamillenöl, Nelkenöl, Melissenöl,Minzöl, Zimtblaetteröl, Lindenbluetenöl, Wacholderbeeröl, Vetiveröl, Olibanumöl, Galbanumöl und Labdanumöl sowie Orangenblütenöl, Neroliöl, Orangenschalenöl und Sandelholzöl. Ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limette-öl, Mandarinenoel, Melissenöl, Moschuskerneröl, Myrrhenöl, Nelkenöl, Neroliöl, Niaouliöl, Olibanumöl, Origanumöl, Palmarosaöl, Patchuliöl,Perubalsamöl, Petitgrainöl, Pfefferöl, Pfefferminzöl, Pimentöl, Pine-öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-öl, Y-sop-öl, Zimtöl, Zimtblätteröl, Zitronellöl, Zitronenöl sowie Zypressenöl.

Ebenfalls als Duftstoff geeignet sind sogenannte Duftstoffvorläufer (Pro-Drug). Bei dieser Klasse von Verbindungen handelt es sich um Verbindungen, welche durch das Aufbrechen einer chemischen Bindung, beispielsweise durch Hydrolyse, ein erwünschtes Geruchs- und/oder Duftstoffmolekül freisetzt. Typischerweise wird zur Bildung eines Duftstoffvorläufers ein gewünschtes Duftstoffrohmaterial chemisch mit einem Träger, vorzugsweise einem geringfügig flüchtigen oder mäßig flüchtigen Träger, verbunden. Die Kombination führt zu einem weniger flüchtigen und stärker hydrophoben Duftstoffvorläufer mit verbesserter Anlagerung auf Stoffen. Der Duftstoff wird danach durch Aufbrechen der Bindung zwischen dem Duftstoffrohmaterial und dem Träger freigesetzt, beispielsweise durch eine Veränderung des pH-Werts (z. B. durch Transpiration beim Tragen), Luftfeuchtigkeit, Wärme und/oder Sonnenlicht während der Lagerung oder des Trocknens auf der Wäscheleine.

Das Duftstoffrohmaterial für Verwendung in Duftstoffvorläufern sind typischerweise gesättigte oder ungesättigte, flüchtige Verbindungen, die einen Alkohol, einen Aldehyd und/oder eine Ketongruppe enthalten. Zu den hierin nützlichen Duftstoffrohmaterialien gehören jegliche wohlriechenden Substanzen oder Mischungen von Substanzen, die bereits oben beschrieben wurden.

Besondere vorteilhafte, einsetzbare Duftstoffvorläufer gehorchen der Formel (III)

R-C(OR¹)(OR²)-OR³ (III)

worin R Wasserstoff, lineares C₁-C₈-Alkyl, verzweigtes C₃-C₂₀-Alkyl, cyclisches C₃-C₂₀-Alkyl, verzweigtes cyclisches C₆-C₂₀-Alkyl, lineares C₆-C₂₀-Alkenyl, verzweigtes C₆-C₂₀-Alkenyl, cyclisches C₆-C₂₀-Alkenyl, verzweigtes cyclisches C₆-C₂₀-Alkenyl, substituiertes oder unsubstituiertes C₆-C₂₀-Aryl und Mischungen hiervon bedeutet; R¹, R² und R³ unabhängig lineares, verzweigtes oder substituiertes C₁-C₂₀-Alkyl; lineares, verzweigtes oder substituiertes C₂-C₂₀-Alkenyl; substituiertes oder unsubstituiertes, cyclisches C₃-C₂₀-Alkyl; substituiertes oder substituiertes C₆-C₂₀-Aryl, substituiertes oder unsubstituiertes C₂-C₄₀-Alkylenoxy; substituiertes oder unsubstituiertes C₃-C₄₀-Alkylenoxyalkyl; substituiertes oder unsubstituiertes C₆-C₄₀-Alkylenaryl; substituiertes oder unsubstituiertes C₆-C₃₂-Ary!oxy; substituiertes oder unsubstituiertes C₆-C₄₀-Alkylenoxyaryl; C₆-C₄₀-Oxyalkylenaryl und Mischungen hiervon bedeuten. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer, sind Acetale oder Ketale, vorzugsweise gehorchend der Formel (IV)

R-C(R¹)(OR³)-OR2 (IV)

worin R lineares C₁-C₂₀-Alkyl, verzweigtes C₃-C₂₀-Alcyl, cyclisches C₆-C₂₀-Alkyl, verzweigtes cyclisches C₆-C₂₀-Alkyl, lineares C₂-C₂₀-Alkenyl, verzweigtes C₃-C₂₀-Alkenyl, cyclisches C6-C20-Alkenyl, verzweigtes cyclisches C₆-C₂₀-Alkenyl, substituiertes oder unsubstituiertes C₆-C₂₀-Aryl und Mischungen hiervon ist; R¹ Wasserstoff oder R ist; R² und R³ jeweils unabhängig voneinander gewählt sind aus der Gruppe, bestehend aus lineares C₁-C₂₀-Alkyl, verzweigtes C₃-C₂₀-Alkyl, cyclisches C₃-C₂₀-Alkyl, verzweigtes cyclisches C₆-C₂₀-Alkyl, lineares C₆-C₂₀-Alkenyl, verzweigtes C₆-C₂₀-Alkenyl, cyclisches C₆-C₂₀-Alkenyl, verzweigtes cyclisches C₆-C₂₀-Alkenyl, C₆-C₂₀-Aryl, substituiertes C₇-C₂₀-Aryl und Mischungen hiervon. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer gehorchen der Formel (V)

R⁴O-C(OR¹)(OR³)-OR² (V)

worin R¹, R², R³ und R⁴ unabhängig voneinander lineares, verzweigtes oder substituiertes C₁-C₂₀-Alkyl; lineares, verzweigtes oder subsituiertes C₂-C₂₀-Alkenyl; substituiertes oder unsubstituiertes, cyclisches C₅-C₂₀-Alkyl; substituiertes oder unsubstituiertes C₆-C₂₀-Aryl, substituiertes oder unsubstituiertes C₂-C₄₀-Alkylenoxy; substituiertes oder unsubstituiertes C₃-C₄₀-Alkylenoxyalkyl; substituiertes oder unsubstituiertes C₆-C₄₀-Alkylenaryl; substituiertes oder unsubstituiertes C₆-C₃₂-Aryloxy; substituiertes oder unsubstituiertes C₆-C₄₀-Alkylenoxyaryl; C₆-C₄₀-Oxyalkylenaryl; und Mischungen hiervon sind. Der Einsatz solcher Substanzen, insbesondere in (vorzugsweise wasserunlöslichen) Mikrokapseln, entspricht einer bevorzugten Ausführungsform der Erfindung.

Besonders bevorzugt ist es, wenn die eingesetzten Riechstoffe Kieselsäureester-Mischungen umfassen. Kieselsäureester werden beispielsweise durch die Formel (V)

R-(-O-Si(OR)₂-)ₙ-OR (V)

beschrieben, wobei R unabhängig voneinander ausgewählt ist aus der Gruppe, die H, die geradkettigen oder verzweigten, gesättigten oder ungesättigten, substituierten oder unsubstituierten C₁-C₆-Kohlenwasserstoffreste und die Duftstoffalkoholreste und/oder Biozidalkoholreste enthält, und m Werte aus dem Bereich 1 bis 20 und n Werte aus dem Bereich 2 bis 100 annimmt. Vorzugsweise enthalten die Kieselsäureester der Formeln zumindest einen Duftstoffalkoholrest und/oder Biozidalkoholrest.

Die Kieselsäureester-Mischungen können verkapselt, aber auch unverkapselt zum Einsatz kommen. Die Anwesenheit von Kieselsäureester-Mischungen führt oftmals dazu, dass der erzielbare Dufteindruck, sowohl was Gefallen als auch Intensität anbetrifft, noch weiter verbessert werden kann. Der Dufteindruck ist nicht nur qualitativ, d. h. das Gefallen anbetreffend, besser, sondern halt auch länger an.

Die Kieselsäureester-Mischungen können auch in den Mikrokapseln enthalten sein. Wenn die Kieselsäureester-Mischungen in den Mikrokapseln vorzugsweise mindestens 2 Gew.- % der gesamten verkapselten Riechstoffmenge ausmachen, Gew.- % bezogen auf die Menge der verkapselten Riechstoffe, so liegt eine bevorzugte Ausführungsform der Erfindung vor, welche eine weitere Verbesserung des angestrebten Wohlgeruchseffektes nach dem Trocknen bewirkt.

Besonders geeignete Duftstoffvorläufer sind Reaktionsprodukte von Verbindungen, die mindestens eine primäre und/oder sekundäre Amingruppe umfassen, beispielsweise einem aminofunktionellen Polymer, insbesondere einem aminofunktionellen Silikon, und einem Duftstoffbestandteil, der aus Keton, Aldehyd und Mischungen davon ausgewählt ist. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Aromastoffe umfassen beispielsweise: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Bevorzugt werden Mischungen verschiedener Duftstoffe (aus den verschiedenen oben genannten Duftstoffklassen) verwendet, die gemeinsam eine ansprechende Duftnote erzeugen. In diesem Fall ist die Gesamtmenge des mindestens einen Duftstoffs die Menge aller Duftstoffe in der Mischung zusammen bezogen auf die Gesamtmenge des Mittels.

### FARBSTOFFE

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbtoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Ebenfalls geeignet sind die bekannten zugelassenen Lebensmittelfarbstoffe:

| | | |
|---|---|---|
| Allurarot AC | E 129 | rot |
| Aluminium | E 173 | silbrig-grau |
| Amaranth | E 123 | rot |
| Anthocyane | E 163 | violett, blau |
| Azorubin | E 122 | rot |
| Betanin | E 162 | rot |
| Braun FK | E 154 | gelb-braun |
| Braun HT | E 155 | rot-braun |
| Brillantblau FCF | E 133 | blau |
| Brillantschwarz BN | E 151 | violett, braun, schwarz |
| Calciumcarbonat | E 170 | |
| Canthaxanthin | E 161 g | |
| Carotin | E 160 a | |
| * Annatto (Norbixin) | E 160 b | |
| * Capsanthin | E 160 c | |
| * Lycopin | E 160 d | |
| * 8'-Apo-β-caroten-8'-al | E 160 e | |
| * Ethyl-8'-apo-β-caroten-8'-oat | E 160 f | |
| Chinolingelb | E 104 | |
| Chlorophyll | E 140 | grün |
| Cochenillerot A | E 124 | |
| Curcumin | E 100 | |
| Eisenoxid | E 172 | |
| Erythrosin | E 127 | |
| Gelborange S | E 110 | |
| Gold | E 175 | |
| Grün S | E 142 | |
| Indigotin | E 132 | |
| Koschenille | E 120 | |
| Kupferhaltige Komplexe der | | |
| Chlorophylle und Chlorophylline | E 141 | |
| Lactoflavin | E 101 | |
| Litholrubin BK | E 180 | |
| Lutein | E 161 b | |
| Patentblau V | E 131 | |
| Pflanzenkohle | E 153 | |
| Riboflavin (Vitamin B2) | E 101 | |
| * Riboflavin-5-phosphat | E 101 a | |
| Saflor | kirschrot bis braungelb | |
| Silber | E 174 | |
| Tartrazin | E 102 | Zitronengelb |
| Titandioxid | E 171 | |
| Zuckerkulör | E 150 a | |
| * Sulfitlaugen-Zuckerkulör | E 150 b | |
| * Ammoniak-Zuckerkulör | E 150 c | |
| * Ammonsulfit-Zuckerkulör | E 150 d | |
| Zeaxanthin | E 161 h | |

Abzugrenzen von den Farbstoffen sind färbende Lebensmittel, die ebenfalls hier eingesetzt werden können. Im Gegensatz zu den Farbstoffen, deren genaue Zusammensetzung gesetzlich vorgeschrieben ist und die durch behördliche Einrichtungen bezüglich ihrer gesundheitlichen Auswirkungen auf den menschlichen Körper sicherheitsbewertet wurden, kommen vermehrt Extrakte von Lebensmitteln zum Einsatz, die eine färbende Wirkung haben. Beispiele sind z.B. Spinatextrakt (grüne Nudeln, Pistazieneis), Rote-Bete-Extrakt, Kurkumin-Extrakt etc.

### GEWERBLICHE ANWENDBARKEIT

Die erfindungsgemäßen Kapseln sind in so gut wie jedem Bereich einsetzbar, insbsondere in Bereichen, in denen Wirkstoffe und Wirkstoffsubstanzen stabilisiert werden sollen, um diese in eine Zusammensetzung einzuformulieren, damit sie später kontrolliert, über einem bestimmten Zeitraum freizusetzen.

Insbesondere sind die erfindungsgemäßen Kapseln geeignet um Wirkstoffe und Wirsubstanzen in kosmetischen Produkten, pharmazeutischen Mitteln oder Nahrungs- und Genussmitteln einzubringen und zu stabilisieren. Insbesondere finden sie Verwendung bei der Herstellung von pharmazeutischen Mitteln oder kosmetischen Produkten zur Anwendung auf der Haut oder zur oralen Verwendung.

Die erfindungsgemäßen Kapseln sind vorteilhaft, da sie eine hohe Beladung aufweisen können und jegliche Art von Wirkstoffen und Wirksubstanzen einschließen können. Insbesondere sind die erfindungsgemäßen Kapseln im Lebensmittelbereich einsetzbar, da sie toxikologisch unbedenklich sind.

### BEISPIELE

### Beispiel 1

### Herstellung von Alginat basierende Kapseln

Alginat und Gummi Arabikum werden in auf 40°C temperierten Wassermenge unter rühren vollständig gelöst. In diese Mischung wird Weizenfaser zugegeben und homogen verteilt. Sollte eine Färbung erwünscht sein, wird die Farbe zunächst in dem Aroma gelöst. Dann wird das Aroma der zuvor hergestellten Mischung zugesetzt und homogen dispergiert (Ultra Turrax ® oder Homogenisator). Die Mischung wird in ein auf 7-10°C temperiertes Calcium-Härtungsbad getropft. Die resultierenden Kapseln werden abgesiebt, gewaschen und in der Wirbelschicht getrocknet.

Es wurden sowohl Alginat als auch Pektin basierende Kapseln wie oben beschrieben hergestellt, in denen ein Wirkstoff (z.B. Aroma) eingekapselt wurde.

Es wurde einmal ein amidiertes Pektin verwendet, welches genau wie das Alginat in vergleichbaren Rezepturen eingesetzt werden konnte.

Weiterhin wurden Mischungen aus niedrigveresterten Pektinen (z.B. Citruspektin, Apfel und sonstige niedrigveresterte Pektin) mit Alginaten untersucht, wobei ein Verhältnis von Alginat zu Pektin im Bereich von 1:2 bis 2:1 verwendet wurde.

Ferner wurden Alginat-Gellan basierende Kapseln wie nach obigen Vorschrift hergestellt, wobei das Verhältnis von Alginat zu Gellan im Bereich von 3:2 bis 3:1 ist.

**Tabelle 1**

| Alginat-Gellan Gummi basierende Kapseln | | | | |
|---|---|---|---|---|
| **Komponenten** | **K1** | **K2** | **K3** | **K4** |
| | **Gew.%** | **Gew.%** | **Gew.%** | **Gew**.% |
| Alginat | 1.03 | 1.18 | - | 0,68 |
| Pektin | - | - | 1.33 | 0,65 |
| G. Arabicum | 0.65 | 0.74 | 0.83 | 0,83 |
| Weizenfaser | 0.56 | 0.64 | 0.77 | 0,77 |
| Aroma | 6.89 | 7.88 | 8.82 | 8,82 |
| Farbstoff | 0.02 | 0.02 | 0.02 | 0.02 |
| Gellan Gummi | 0.37 | 0.84 | - | - |
| Wasser | Add to 100 | Add to 100 | Add to 100 | Add to 100 |
| ***Beladung*** | ***Ca. 75%*** | ***Ca. 75%*** | ***Ca. 75%*** | ***Ca. 75%*** |

Die resultierenden Alginat-Gellan Gummi Kapseln weisen eine durchschnittliche Größe von 925 bis 955 µm auf, bei einer Verwendung von einer Düse von 0.5 mm, mit Temp.Dispersion 40 °C, Temp.Cal.Bad 10 -15 °C, Druck Dispersion 0,19 bar, Frequenz 250 Hz, Amplitude 0,75 A, Temp. Härtungsbad 7-10°C, Durchflußmenge 0,8 kg/h und einer Trocknung mit Silizumd.103846 ca 10% und Trocknung Aeromatic bis 45°C ca 35 min bis 45 min.

Die Verhältnisse der Komponenten in der Standardrezeptur eines Gelkügelchens vor und nach der Trocknung ist bei Alginatkapseln:

**Tabelle 3**

| Verhältnis der einzelnen Komponenten in einer Alginatkapsel | | |
|---|---|---|
| **Komponente** | **Vor der Trocknung** | **Nach der Trocknung** |
| | Gew.% | Gew.% |
| Wasser | 88,231 | - |
| Aroma | 8,823 | 75,11 |
| Natriumalginat E401 high viscous | 1,325 | 11,28 |
| Gummi Arabicum Typ SEYAL | 0,827 | 7,00 |
| Weizenfaser WF 600-30 | 0,772 | 6,57 |

### Beispiel 2

### Herstellung einer O/W-Emulsion

Mischung A und B wurden separat auf 80°C erhitzt, und anschließend Mischung B in Mischung A dispergiert. Dann wird Mischung C zum Gemisch AB dazugegeben und emulgiert mittels eines Ultra Turrax Stirrer. (3 min). Anschließend wurde mit D neutralisiert und abgekühlt. Die erfindungsgemäßen Kaspeln (E) werden in die Emulsion E1 und E2 eingearbeitet und können so getestet werden

**Tabelle 2**

| O/W Emulsionen in denen die erfindungsgemäßen Kapseln eingearbeitet werden | | | | |
|---|---|---|---|---|
| **Ro hmaterial** | | **INCI** | **E1** | **E2** |
| | | | **w**/**w** % | **w**/**w** % |
| **A.** | Emulsiphos | Potassium Cetyl Phosphate, Hydrogenated Palm Glycerides | 2,0 | 2,0 |
| | Cutina PES | Pentaerythrityl Distearate | 2,0 | 2,0 |
| | PCL Solid | Stearyl Heptanoate, Stearyl Caprylate | 2,0 | 2,0 |
| | Lanette O | Cetearyl Alcohol | 2,5 | 2,5 |
| | PCL Liquid 100 | Cetearyl Ethylhexanoate | 5,0 | 5,0 |
| | Isodragol | Triisononanoin | 2,0 | 2,0 |
| | Dow Corning 246 Fluid | Cyclohexasiloxane, Cyclopentasiloxane | 2,0 | 2,0 |
| | Dragoxat 89 | Ethylhexyl Isononanoate | 3,0 | 3,0 |
| **B.** | Ultrez-10 | Carbomer | 0,2 | 0,2 |
| | Keltrol CG | Xanthan Gum | 0,15 | 0,15 |
| **C.** | Wasser | Water (Aqua) | 71,05 | 71,15 |
| | EDTA BD | Disodium EDTA | 0,1 | - |
| | Hydrolite-5 | Pentylene Glycol | 2,0 | 2,0 |
| | Propylene Glycol | Propylene Glycol | 2,0 | 2,0 |
| | SymDiol 68 | 1,2 Hexanediol, Caprylyl Glycol | 0,6 | 0,6 |
| | Glycerin 85% | Glycerin | 2,0 | 2,0 |
| **D.** | Sodium Hydroxide 10% sol. | Sodium Hydroxide | 0,4 | 0,4 |
| **E.** | Erfindungsgemäße Kapseln K2 | (75% active) | 1,0 | 1,0 |

### Beispiel 3

### Lagertests

Die Emulsionen aus Beispiel 2, welche die erfindungsgemäßen Kapseln enthalten wurden bei unterschiedlichen Temperaturen gelagert und visuell und mikroskopisch untersucht.

**Tabelle 3**

| Lagertestergebnisse | | | | | | |
|---|---|---|---|---|---|---|
| **Emulsion** | **T = 5 °C** | **T = RT** | | **T = 40 °C** | | |
| | 3 Monate | 2 Tage | 3 Monate | 1 Woche | 2 Woche | 3 Monate |
| E1 | - | Alle Kapseln abgebaut | - | - | - | - |
| E2 | stabil | stabil | stabil | stabil | stabil | Kapsel sindleicht aufgequollen, dennoch als Partikel noch erkennbar |

Die Emulsion 1 mit EDTA wies nach 2 Tagen bei RT keine Kapseln mehr auf. Alle Kapseln waren nach 2 Tagen zerstört und in der Emulsion aufgelöst. Die Emulsion 2 zeigte eine bessere Stabilität bei unterschiedlichen Lagertemeraturen. Sebst nach 3 Monaten waren noch Kapseln in der Emulsion visuell sichtbar und mikroskopisch nachweisbar, auch wenn die Kapseln nach 3 Monaten Lagerung leicht aufgequollen waren. Wurde die Emulsion auf der Haut verrieben, so lösten sich die Kapseln auf und setzten den Wirkstoff frei.

### Beispiel 4

### Lagertests in Produkten

Die erfindungsgemäßen getrockneten Kapseln wurden in Zahnpasta, Margarine und Hautcreme eingearbeitet und nach 3 Monaten bei Raumtemperatur gelagert.

Die getrockneten Kapseln sind nach 3 Monaten stabil und zeigten keinerlei Veränderungen. Bei den Kapseln, die in Zahnpasta eingearbeitet wurden, waren die Kapseln, die auf Alginat- oder Pektinbasis aufgebaut waren, stark aufgequollen, die Kapselstruktur ist jedoch noch erkennbar geblieben. Bei Kapseln auf Alginat-Gellan Gummi Basis zeigte sich eine verbesserte Stabilität. Diese Kapseln waren nach 1 Monat Lagerung bei RT noch stabil.

Bei der Margarine und der Hautcreme waren die Kapseln auch nach 3 Monaten noch stabil.

### Beispiel 5

### Formulierungsbeispiele A bis G

### Kaugummimasse A-C

Die erfindungsgemäßen Kapseln, wurden in einer Kaugimmimasse gemäß Tabelle 4 eingearbeitet.

**Tabelle 4**

| Kaugummimassen A-C | | | |
|---|---|---|---|
| **Zusammensetzung** | **A** | **B** | **C** |
| Polyisobutylen (MW 20.000) | 20.0 | 25.0 | 30.0 |
| Sorbitol | 51,0 | 47,5 | 44,5 |
| Mannitol | 5,0 | 4,3 | 3,6 |
| Glycerin | 8,0 | 8,0 | 7,0 |
| Lycasin:Glycerin (1:1) | 8,2 | 8,0 | 7,0 |
| Lecithin | 0,2 | 0,2 | 0,2 |
| Aromamischung | 1,0 | 1,0 | 1,0 |
| Wasser | Ad 100 | | |

### Kosmetische Zusammensetzung D-G , enthaltend erfindungsgemäße Kapseln

In D und E wurden Aromen in den erfindungsgemäßen Kapseln eingearbeitet.

In Cremes F und G wurden Kühlwirkstoffe in den erfindungsgemäßen Kapseln eingearbeitet.
D = Badelotion, E = Softcreme, F, G = Feuchtigkeitscreme

**Tabelle 5**

| Beispielformulierungen D-G einer kosmetischen Zubereitung | | | | | |
|---|---|---|---|---|---|
| **Komponenten (INCI)** | | **D** | **E** | **F** | **G** |
| **Texapon® NSO** | | - | - | - | - |
| Sodium Laureth Sulfate | | | | | |
| **Plantacare® 818** | | - | - | - | - |
| Coco Glucosides | | | | | |
| **Plantacare® PS 10** | | 22,0 | - | - | - |
| Sodium Laureth Sulfate (and) Coco Glucosides | | | | | |
| **Dehyton® PK 45** | | 15,0 | - | - | - |
| Cocamidopropyl Betaine | | | | | |
| **Emulgade® SE** | | - | 5,0 | 5,0 | 4,0 |
| Glyceryl Sterate (and) Ceteareth 12/20 (and) Cetearyl Alcohol (and) Cetyl Palmitate | | | | | |
| **Eumulgin® B1** | | - | - | - | 1,0 |
| Ceteareth-12 | | | | | |
| **Lameform® TGI** | | - | - | - | - |
| **Polyglyceryl-3 Isostearate** | | | | | |
| **Dehymuls® PGPH** | | - | - | - | - |
| **Polyglyceryl-2 Dipolyhydroxystearate** | | | | | |
| **Monomuls® 90-O 18** | | - | - | - | - |
| **Glyceryl Oleate** | | | | | |
| **Cetiol® HE** | | 2,0 | - | - | - |
| **PEG-7 Glyceryl Cocoate** | | | | | |
| **Cetiol® OE** | | - | - | - | - |
| **Dicaprylyl Ether** | | | | | |
| **Cetiol® PGL** | | - | - | - | 3,0 |
| **Hexyldecanol (and) Hexyldecyl Laurate** | | | | | |
| **Cetiol® SN** | | 1.0 | 3,0 | 3,0 | - |
| Cetearyl Isononanoate | | | | | |
| **Cetiol® V** | | - | 3,0 | 3,0 | - |
| Decyl Oleate | | | | | |
| **Myritol® 318** | | - | - | - | 3,0 |
| Coco Caprylate Caprate | | | | | |
| **Bees Wax** | | - | - | - | - |
| **Nutrilan® Elastin E20** | | - | 2,0 | - | - |
| Hydrolyzed Elastin | | | | | |
| **Nutrilan® I-50** | | - | - | 2,0 | - |
| Hydrolyzed Collagen | | | | | |
| **Gluadin® AGP** | | 0,5 | - | - | 0,5 |
| Hydrolyzed Wheat Gluten | | | | | |
| **Gluadin® WK** | | 2,0 | - | - | - |
| Sodium Cocoyl Hydrolyzed Wheat Protein | | | | | |
| **Euperlan® PK 3000 AM** | | 5,0 | - | - | - |
| Glycol Distearate (and) Laureth-4 (and) Cocamidopropyl Betaine | | | | | |
| **Arlypon® F** | | - | - | - | - |
| Laureth-2 | | | | | |
| **Hydagen® CMF** | | 1,0 | 1,0 | 1,0 | 1,0 |
| Chitosan | | | | | |
| **Erfindungsgemäße Kapseln** | | 1.0 | 1.0 | 1.0 | 1.0 |
| **Glycerin** (86 Gew.%ig) | | - | 3,0 | 3,0 | 5,0 |
| **Water** | **add to** | 100 | 100 | 100 | 100 |

## Patentansprüche

1. Kapseln, umfassend
(a) mindestens eine gelierbare Substanz,
(b) mindestens einen Emulgator,
(c) mindestens einen Füllstoff und
(d) mindestens einen Wirkstoff, welcher verkapselt werden soll,
wobei
(i) die gelierbare Substanz (a) ausgewählt ist aus Alginat und/oder Pektin und/oder Gellan Gummi,
(ii) der Emulgator (b) ausgewählt ist aus der Gruppe bestehend aus Polysorbate, Zuckereestern, Saponine, Gummi Arabicum, modifiziertem Gummi Arabicum und/oder modifizierte Stärke,
(iii) der Füllstoff (c) ausgewählt ist aus der Gruppe bestehend aus pflanzlichen Fasern, mikrokristalline Cellulose, Silicagele, native Stärke und/oder Silikate, und
(iv) der Wirkstoff (d) in einer Emulsion umfassend die gelierbare Substanz (a), den Emulgator (b) und den Füllstoff (c) dispergiert vorliegt.

2. Kapseln nach [Anspruch 1, **dadurch gekennzeichnet, dass** das Verhältnis von Alginat zu Pektin von 1:2 bis 2:1 bei einem Gemisch aus Alginat und Pektin und das Verhältnis von Alginat zu Gellan Gummi von 3:2 bis 3:1 bei einem Gemisch von Alginat mit Gellan Gummi, ist.

3. Kapseln nach den Ansprüchen 1 und/oder 2, **dadurch gekennzeichnet, dass** der Füllstoff eine pflanzliche Faser ist, welche ausgewählt ist aus der Gruppe bestehend aus Apfelfasern, Bambusfasern, Haferfasern, Erbenfasern, Kartoffelfasern und/oder Weizenfasern.

4. Kapseln nach mindestens einem der Ansprüche 1 bis 3, **dadurch gekennzeichnet, dass** der Wirkstoff oder die Wirksubstanz ausgewählt ist aus der Gruppe bestehend aus Aromastoffen, Duftstoffen, Nahrungsergänzungsmittelsubstanzen, wie beispielsweise Vitamine, Mineralstoffe, Antioxidantien, Anthocyane, Coenzym10 etc., sowie kosmetischen Wirksubstanzen und / oder pharmazeutischen Wirksubstanzen.

5. Kapseln nach mindestens einem der Ansprüche 1 bis 4, **dadurch gekennzeichnet, dass** das Verhältnis von Emulgator zu Füllstoff im Bereich von 2:1 bis 1:2 vorliegt.

6. Kapseln nach mindestens einem der Ansprüche 1 bis 5, **dadurch gekennzeichnet, dass** die Wirkstoffbeladung in den Kapseln zwischen 20 bis 95 Gew.-% liegt.

7. Kapseln nach mindestens einem der Ansprüche 1 bis 6, **dadurch gekennzeichnet, dass** die Kapseln einen mittleren Durchschnittsdurchmesser von 200 bis 1500 µm aufweisen.

8. Kapseln nach einem der Ansprüche 1 bis 7 zur Verwendung in kosmetischen Produkten, pharmazeutischen Mitteln oder in Nahrungs- und Genussmittel.

9. Verfahren zur Herstellung von Kapseln nach einem der vorherigen Ansprüche 1 bis 8, umfassend
(v) das Bilden einer Mischung aus a) mindestens einer gelierbaren Substanz, b) mindestens einem Emulgator, c) mindestens einem Füllstoffen und d) mindesten einem Wirkstoff oder Wirksubstanz,
(vi) das Einbringen von Tropfen der Mischung aus v) in eine Lösung aus einem multivalenten Kation, so dass eine Vernetzung stattfindet und eine Gelierung erfolgt,
(vii) die Trennung der gebildeten Kapseln aus Schritt vi)
(viii)das Trocknen der erhaltenen Kapseln aus Schritt vii).

10. Verwendung von Kapseln nach einem der Ansprüche 1 bis 8 zur Herstellung von kosmetischen Produkten, pharmazeutischen Mitteln oder Nahrungs- und Genussmitteln.

11. Verwendung von Kapseln nach einem der Ansprüche 1 bis 8 zur Herstellung von pharmazeutischen Mitteln oder kosmetischen Produkten zur Anwendung auf der Haut oder zur oralen Verwendung.

12. Verwendung von Kapseln nach einem der Ansprüche 1 bis 8 zur kontrollierten, verlängerten und verzögerten Abgabe und Freisetzung von Wirkstoffen und Wirksubstanzen.

13. Kapseln erhältlich durch die Schritte, umfassend:
(i) das Bilden einer Mischung aus
(a) mindestens einer gelierbaren Substanz,
(b) mindestens einem Emulgator,
(c) mindestens einem Füllstoffen und
(d) mindesten einem Wirkstoff oder Wirksubstanz,
(ii) das Einbringen von Tropfen der Mischung aus (i) in eine Lösung aus einem multivalenten Kation, so dass eine Vernetzung stattfindet und eine Gelierung erfolgt,
(iii) die Trennung der gebildeten Kapseln aus Schritt (ii),
(iv) das Trocknen der erhaltenen Kapseln aus Schritt (iii),
wobei
(v) die gelierbare Substanz (a) ausgewählt ist aus der der Gruppe bestehend aus Alginat und/oder Pektin und/oder Gellan Gummi,
(vi) der Emulgator (b) ausgewählt ist aus der Gruppe bestehend aus Polysorbaten, Zukkereestern, Saponinen, Gummi Arabicum und/oder modifizierte r Stärke, und
(vii) der Füllstoff (c) ausgewählt ist aus der Gruppe bestehend aus pflanzlichen Fasern, mikrokristalliner Cellulosen, Silicagelen, nativen Stärken und/oder Silikaten.

## Claims

1. Capsules, encompassing
(a) at least one gel forming substance,
(b) at least one emulsifier,
(c) at least one filler, and
(d) at least one active for encapsulation,
whereby
(i) said gel-forming substance (a) is selected from the group consisting of alginate and/or pectin and/or gellan gum;
(ii) said emulsifier (b) is selected from the group consisting of polysorbates, sugar esters, saponins, gum Arabic, modified gum Arabic and/or modified starch;
(iii) said filler (c) is selected from the group consisting of vegetable fibres, microcrystalline cellulose, silica gels, native starch and/or silicates; and
(iv) said active (d) is dispersed in an emulsion comprising said gel-forming substance (a), said emulsifier (b) and said filler (c)

2. Capsules according to Claim 1, **characterised in that** the in case of a mixture of alginate and pectin the ratio of alginate and pectin is 1:2 to 2:1 and in case of a mixture of alginate and gellan gum the ratio of alginate and gellan gum is 3:2 to 2:3.

3. Capsules according to Claims 1 and/or 2, **characterised in that** the filler represents a vegetable fibre selected from the group consisting of apple fibres, bamboo fibres, oat fibres, pea fibres, potato fibres and/or wheat fibres.

4. Capsules according to any of Claims 1 to 3, **characterised in that** said active or said actives are selected from the group consisting of aroma compounds, fragrances, and dietary supplements as for example vitamins, minerals, anti-oxidants, anthocyans, coenzyme 10 etc. and cosmetic actives and/or pharmaceutical actives.

5. Capsules according to any of Claims 1 to 4, **characterised in that** emulsifiers and fillers are present in a ratio of 2:1 to 1:2.

6. Capsules according to any of Claims 1 to 5, **characterised in that** the active load of said capsules ranges from 20 to 95 wt.-%.

7. Capsules according to any of Claims 1 to 6, **characterised in that** said capsules show an average diameter of from 200 to 1,500 µm.

8. Capsules according to any of Claims 1 to 7 for use in cosmetic products, pharmaceutical products or products for alimentation or pleasure.

9. Process for the manufacture of capsules according to any of the preceding Claims 1 to 8, encompassing:
(v) forming a mixture of (a) at least one gel-forming substance, (b) at least one emulsifier, (c) at least one filler and (d) at least one active or active ingredient;
(vi) introducing droplets of the mixture of (v) into a solution of a multivalent cation to achieve cross-linking and formation of a gel;
(vii) separating-off the capsules thus obtained from step (vi)
(viii) drying of the capsules thus obtained from step (vii).

10. Use of capsules according to Claims 1 to 8 for the manufacture of cosmetic products, pharmaceutical products or products for alimentation or pleasure.

11. Use of capsules according to Claims 1 to 8 for the manufacture of pharmaceutical products for application on skin or for oral uptake.

12. Use of capsules according to Claims 1 to 8 for controlled, prolonged and retarded release und liberation of actives and active ingredients.

13. Capsules obtainable by the steps encompassing:
(i) forming a mixture of
(a) at least one gel-forming substance,
(b) at least one emulsifier,
(c) at least one filler and
(d) at least one active or active ingredient,
(ii) introducing droplets of the mixture of (i) into a solution of a multivalent cation to achieve cross-linking and formation of a gel;
(iii) separating off the capsules thus obtained from step (ii);
(iv) drying of the capsules thus obtained from step (iii);
whereby
(v) said gel-forming substance (a) is selected from the group consisting of alginate and/or pectin and/or gellan gum;
(vi) said emulsifier (b) is selected from the group consisting of polysorbates, sugar esters, saponins, gum Arabic, modified gum Arabic and/or modified starch; and
(vi) said filler (c) is selected from the group consisting of vegetable fibres, microcrystalline cellulose, silica gels, native starch and/or silicates.

## Revendications

1. Capsules, comprenant
(a) au moins une substance gélifiable,
(b) au moins un émulsifiant,
(c) au moins une charge et
(d) au moins un principe actif qui doit être encapsulé,
dans lesquelles
(i) la substance gélifiable (a) est choisie parmi un alginate et/ou la pectine et/ou la gomme gellane,
(ii) l'émulsifiant (b) est choisi dans le groupe constitué par les polysorbates, les esters de sucres, les saponines, la gomme arabique, une gomme arabique modifiée et/ou un amidon modifié,
(iii) la charge (c) est choisie dans le groupe constitué par les fibres végétales, la cellulose microcristalline, les gels de silice, l'amidon natif et/ou les silicates, et
(iv) le principe actif (d) est présent dispersé dans une émulsion comprenant la substance gélifiable (a), l'émulsifiant (b) et la charge (c).

2. Capsules selon la revendication 1, **caractérisées en ce que** le rapport de l'alginate à la pectine vaut de 1:2 à 2:1 pour un mélange d'alginate et de pectine et le rapport de l'alginate à la gomme gellane vaut de 3:2 à 3:1 pour un mélange d'alginate avec de la gomme gellane.

3. Capsules selon la revendication 1 et/ou la revendication 2, **caractérisées en ce que** la charge est une fibre végétale, qui est choisie dans le groupe constitué par les fibres de pomme, les fibres de bambou, les fibres d'avoine, les fibres de pois, les fibres de pommes de terre et/ou les fibres de blé.

4. Capsules selon au moins l'une quelconque des revendications 1 à 3, **caractérisées en ce que** le principe actif ou la substance active est chois(e) dans le groupe constitué par des arômes, des parfums, des substances qui sont des compliments alimentaires, comme par exemple des vitamines, des substances minérales, des antioxydants, des anthocyanes, la coenzyme 10, etc., ainsi que des substances actives cosmétiques et/ou des substances actives pharmaceutiques.

5. Capsules selon au moins l'une quelconque des revendications 1 à 4, **caractérisées par** le rapport d'émulsifiant à charge se situe dans la plage de 2:1 à 1:2.

6. Capsules selon au moins l'une quelconque des revendications 1 à 5, **caractérisées en ce que** dans les capsules la charge de principe actif est comprise entre 20 et 95 % en poids.

7. Capsules selon au moins l'une quelconque des revendications 1 à 6, **caractérisées en ce que** les capsules présentent en moyenne un diamètre moyen de 200 à 1 500 µm.

8. Capsules selon l'une quelconque des revendications 1 à 7, destinées à l'utilisation dans des produits cosmétiques, des produits pharmaceutiques ou dans des produits alimentaires et autres produits de consommation.

9. Procédé pour la production de capsules selon au moins l'une des revendications précédentes 1 à 8, comprenant
(v) la formation d'un mélange de a) au moins une substance gélifiable, b) au moins un émulsifiant, c) au moins une charge et d) au moins un principe actif ou une substance active,
(vi) l'introduction de gouttes du mélange de v) dans une solution d'un cation multivalent, de sorte qu'une réticulation a lieu et une gélification s'effectue,
(vii) la séparation des capsules formées provenant de l'étape vi)
(viii) le séchage des capsules obtenues provenant de l'étape vii).

10. Utilisation de capsules selon l'une quelconque des revendications 1 à 8 pour la fabrication de produits cosmétiques, de produits pharmaceutiques ou de produits alimentaires et d'autres produits de consommation.

11. Utilisation de capsules selon l'une quelconque des revendications 1 à 8 pour la fabrication de produits pharmaceutiques ou de produits cosmétiques destinés à l'application sur la peau ou à l'emploi par voie orale.

12. Utilisation de capsules selon l'une quelconque des revendications 1 à 8 pour la décharge et la libération programmées, prolongées et retardées de principes actifs et de substances actives.

13. Capsules pouvant être obtenues par les étapes comprenant :
(i) la formation d'un mélange à partir
(a) d'au moins une substance gélifiable,
(b) d'au moins un émulsifiant,
(c) d'au moins une charge et
(d) d'au moins un principe actif ou une substance active,
(ii) l'introduction de gouttes du mélange provenant de (i) dans une solution d'un cation multivalent, de sorte qu'une réticulation a lieu et une gélification s'effectue
(iii) la séparation des capsules formées provenant de l'étape (ii),
(iv) le séchage des capsules obtenues provenant de l'étape (iii),
et dans lesquelles
(v) la substance gélifiable (a) est choisie dans le groupe constitué par un alginate et/ou la pectine et/ou la gomme gellane,
(vi) l'émulsifiant (b) est choisi dans le groupe constitué par les polysorbates, les esters de sucres, les saponines, la gomme arabique et/ou un amidon modifié, et
(viii) la charge (c) est choisie dans le groupe constitué par les fibres végétales, les celluloses microcristallines, les gels de silice, les amidons natifs et/ou les silicates.
